Europäisches Patentamt

European Patent Office

Office européen des brevets

(11) Veröffentlichungsnummer: **0 180 247**
**B1**

(12) **EUROPÄISCHE PATENTSCHRIFT**

(45) Veröffentlichungstag der Patentschrift:
26.07.89

(21) Anmeldenummer: 85113975.8

(22) Anmeldetag: 04.11.85

(51) Int. Cl.⁴: **A 61 C 8/00**

(54) Enossales Implantat zur Befestigung von festsitzendem oder abnehmbarem Zahnersatz.

Teilanmeldung 88110095 eingereicht am 24.06.88.

(30) Priorität: 09.11.84 DE 3440952
26.04.85 DE 3515154
10.09.85 DE 3532125

(43) Veröffentlichungstag der Anmeldung:
07.05.86 Patentblatt 86/19

(45) Bekanntmachung des Hinweises auf die Patenterteilung:
26.07.89 Patentblatt 89/30

(84) Benannte Vertragsstaaten:
AT BE CH DE FR GB IT LI LU NL SE

(56) Entgegenhaltungen:
EP-A- 0 015 599
EP-A- 0 083 028
DE-A- 2 247 721
DE-A- 2 249 051
DE-A- 3 043 336
DE-A- 3 300 764
US-A- 3 934 347

(73) Patentinhaber: IMPLANTO-LOcK Gesellschaft mit beschränkter Haftung für Implantatforschung und Entwicklung, Ness 1, D-2000 Hamburg 11 (DE)

(72) Erfinder: Koch, Werner-Lutz, Loesnweg 7, D-3073 Liebenau/Hann (DE)

(74) Vertreter: Richter, Joachim, Dipl.-Ing. et al, Patentanwälte Richter u.Werdermann Neuer Wall 10, D-2000 Hamburg 36 (DE)

**Beschreibung**

Die Erfindung betrifft ein enossales Implantat zur Befestigung von festsitzendem oder abnehmbarem Zahnersatz, bestehend aus zwei miteinander verbindbaren Teilen, von denen das eine Teil als in den Kieferknochen einbringbarer und sich in diesem kraftschlüssig verankernder Primärzylinder mit einer mittigen Längsbohrung und das andere Teil als Sekundärzylinder ausgebildet ist, der einen in die Längsbohrung des Primärzylinders einbringbaren und in diesem gehaltenen Implantatpfosten zur Aufnahme des Zahnersatzes aufweist, und aus einem an dem Implantatpfosten vorgesehenen Implantataufsatz, wobei der Implantatpfosten an seinem freien oberen Ende für den Anschluss des Zahnersatzes ausgebildet ist und von einem die im Bereich des Zahnersatzes oder im Munde auftretenden Horizontal- und/ oder Vertikal- und/oder Torsions-Kräfte und Schwingungen in den Sekundärzylinder und von dort in den Primärzylinder umleitenden Kraftleitungssystem aus mehreren, sich aneinanderreihenden Bereichen mit unterschiedlichen elastischen Eigenschaften umgeben und in dem Primärzylinder fest oder lösbar gehalten ist.

Durch die EP-A-0 083 028 ist ein Verbindungselement für einen Implantatkörper und eine Suprastruktur mit den Merkmalen des Oberbegriffes des Anspruches 1 bekannt. Bei diesem Verbindungselement besteht der Implantatpfosten aus einem Werkstoff mit einem hohen Elastizitätsmodul und weist eine Verdickung auf, die aus dem gleichen oder einem anderen Werkstoff als der Implantatpfosten besteht. Ausserdem weist das Verbindungselement eine den Implantatpfosten zusammen mit seiner Verdickung umfassende Umhüllung auf, die in ihrer äusseren Form der Bohrung des Implantats entspricht und aus einem Werkstoff besteht, der den gleichen oder einen niedrigeren Elastizitätsmodul aufweist als der Werkstoff, aus dem der Implantatpfosten besteht.

Da die Verdickung des Implantatpfostens bevorzugt aus dem gleichen Werkstoff wie dieser besteht, z.B. aus Metall oder Keramik, und direkt an der Wand der Bohrung des Implantats anliegt, so wie etwa in halber Höhe der Implantatbohrung an dem Implantatpfosten vorgesehen ist, ergibt sich beim Angreifen von Horizontalkräften auf den am freien oberen Ende des Implantatpfostens befestigten Zahnersatzes ein Verschwenken des Implantatpfostens um die Lagerpunkte der Verdickung an der Wandfläche der Implantatbohrung, so dass der Hebeldrehpunkt etwa in der Mitte der Verdickung zu liegen kommt, wenn diese eine kugelförmige Gestalt aufweist. Dadurch ergibt sich zwangsläufig, dass der Implantatpfosten als zweiarmiger Hebel wirkt, so dass ein Ableiten auftretender Kräfte über die Verdickung in den Implantatkörper erfolgt. Da das untere Ende des Implantatpfostens dann frei verschwenkbar ist, ist ein Ableiten auftretender Kräfte in den bodenseitigen Bereich des Implantatkörpers nicht möglich.

Des weiteren ist bei dem bekannten Implantat eine den Implantatpfosten mit seiner Verdickung umfassende Umhüllung vorgesehen, die aus einem Werkstoff mit dem gleichen oder einem niederen Elastizitätsmodul als der Implantatpfosten und seine Verdickung besteht. Als Werkstoff für diese Umhüllung werden geeignete Kunststoffe, wie z.B. Polyäthylen, eingesetzt. Dadurch weist die Umhüllung eine Elastizität auf, und zwar sowohl in den Bereichen oberhalb der Verdickung an dem Implantatpfosten als auch unterhalb dieser Verdickung. Dadurch ist der Implantatpfosten bodenseitig nicht fest in einem Umhüllungsabschnitt aus einem unelastischen Werkstoff eingebunden, so dass auch in diesem Fall ein Ableiten auftretender Spannungskräfte im bodenseitigen Bereich des Implantatkörpers nicht möglich ist, da auftretende Spannungskräfte von der elastischen Umhüllung aufgenommen werden; es kommt somit zu einer Druckabsorption durch den elastischen Werkstoff der Umhüllung. Ausserdem behält der Implantatpfosten seine Wirkung als zweiarmiger Hebel bei, da die Umhüllung in den Bereichen oberhalb und unterhalb der Verdickung aus einem elastischen Werkstoff besteht. Wesentlich ist jedoch bei diesem bekannten Implantat, dass der Implantatpfosten im bodenseitigen Bereich in der Implantatbohrung federnd gelagert ist.

Des weiteren ist das bodenseitige Ende des Implantatpfostens in einer Führungskappe angeordnet, die am Boden der Implantatbohrung angeordnet ist. Diese Führungskappe besteht aus einem Werkstoff mit einem höheren Elastizitätsmodul als der übrige Teil der Umhüllung, so dass letztlich diese Führungskappe eine Elastizität besitzt, die zwar geringer ist als die Elastizität der übrigen Umhüllung, jedoch besteht die Führungskappe nicht aus einem unelastischen Werkstoff, aus dem der Implantatpfosten mit seiner Verdickung besteht. Der Implantatpfosten behält seine Wirkung als zweiarmiger Hebel immer bei, denn bei an der Implantatkrone angreifenden Horizontalkräften verteilen sich diese zu gleichen Teilen auf die Nachbarpfeiler, wobei der für das Implantat verbleibende Anteil in Höhe der Verdickung und nicht im bodenseitigen Bereich des Implantats angreift.

Auch wenn die Umhüllung aus Werkstoffen mit unterschiedlichem Elastizitätsmodul besteht oder wenn die Umhüllung abschnittsweise entfällt, wird ein Ableiten auftretender Spannungskräfte in den Boden des Implantats nicht erreicht, da der Implantatpfosten bodenseitig elastisch gelagert ist.

Nachteilig ist somit bei diesem Implantat, dass der Hebeldrehpunkt des Implantatpfostens etwa in der Mitte des Implantatkörpers liegt, so dass ein Auslockern des Implantats nicht mit Sicherheit vermeidbar ist. Ausserdem ist ein einwandfreies Ableiten auftretender Spannungskräfte in den Aussenbereich des Implantatkörpers nicht gewährleistet, so dass es zu einer Beschädigung und bei hoher horizontaler Druckbelastung des Zahnersatzes zu einem Bruch des Implantatkörpers kommen kann, insbesondere dann, wenn der Implantatkörper aus einem Keramik-Werkstoff besteht.

Die Erfindung löst die Aufgabe, ein aus einem Primärzylinder und einem in diesem gehaltenen Sekundärzylinder bestehendes enossales Implantat zu schaffen, mit dem ein form- und kraftschlüssiger Verbund zum Knochen hin erreicht wird und bei dem eine belastungsfreie Stabilisierung des Primärzylinders sichergestellt ist und das Implantat keinerlei plastischen Verformungen unterliegt und eine wartungsfreie Mechanik aufweist. Des weiteren soll eine feste Verbindung des Zapfens des Sekundärzylinders in der Längsbohrung des Primärzylinders geschaffen werden, ohne dass die schwingende Eigenschaft des Zapfens beeinträchtigt wird, wobei die im Munde auftretenden Horizontal- und/oder Vertikal- und/oder Torsionskräfte in den Boden des Primärzylinders abgeleitet werden sollen.

Diese Aufgabe wird bei einem gattungsgemässen enossalen Implantat erfindungsgemäss durch die im Anspruch 1 gekennzeichneten Merkmale gelöst.

Hiernach ist das Implantat in der Weise ausgebildet, dass der aus einem spröden Werkstoff bestehende Implantatpfosten von die im Bereich des Zahnersatzes durch auf diesen einwirkende Kaumuskelkraft auftretenden Schwingungen bei gleichzeitiger Verlagerung des Hebeldrehpunktes des Implantatpfostens in den unteren Bereich des Sekundärzylinders von dort in den Primärzylinder umleitenden, rohr- oder ringförmigen Modulelementen als Kraftleitungssystem umgeben ist, das somit mehrere, sich aneinanderreihende Bereiche mit unterschiedlichen elastischen Eigenschaften derart aufweist, dass sich an einen bodenseitigen unelastischen Bereich ein Bereich mit geringer Elastizität und sich wiederum an diesen Bereich ein Bereich mit starker Elastizität anschliesst. Der mit dieser Ausgestaltung erreichte Vorteil besteht im folgenden: Die auf den Implantatpfosten mittels einer Fügeverbindung, z.B. kraftschlüssige Verbindung mit anaeroben Kunststoffen, aufgesetzten Modulelemente bilden ein Kraftleitungssystem mit unterschiedlichen elastischen Eigenschaften. Von den aufeinandergesetzten Modulelementen weist das bodenseitige Modulelement keine Elastizität auf; es ist wie der Implantatpfosten starr ausgebildet. Das auf das bodenseitige Modulelement aufgesetzte mittlere Modulelement besteht aus einem Werkstoff mit geringer Elastizität, während das obere Modulelement aus einem stark elastischen Werkstoff besteht, so dass bei der Einwirkung von Kaumuskelkräften, z.B. horizontal einwirkenden Kräften, auf den Zahnersatz, der Drehpunkt des als Hebel wirkenden Implantatpfostens in den unteren Bereich des Primärzylinders bzw. Implantatkörpers verlegt wird.

Der in dem Sekundärzylinder angeordnete stabförmige Implantatpfosten besteht aus einem spröden Werkstoff, wie z.B. chirurgischem Stahl, und bildet einen Hebel, dessen Drehpunkt aufgrund des speziell ausgebildeten Kraftleitungssystems in das untere Drittel des Primärzylinders verlagert wird. Dadurch, dass der Implantatpfosten von Modulelementen, z.B. Modulrohren, Modulringen u.dgl., umgeben ist, die aus Werkstoffen unterschiedlicher Elastizität bestehen, werden auftretende Schwingungen am Implantatpfosten bei z.B. senkrecht zur Implantatachse einwirkenden Kaukräften abgefangen, vom Kraftleitungssystem aufgenommen und in den unteren Bereich des Primärzylinders umgeleitet. Die Modulelemente fangen somit die Kräfte ab bzw. leiten diese in den Bereich des Drehpunktes um, also in die Tiefe des Implantats bzw. des Primärzylinders, ohne sich dabei zu deformieren oder plastisch zu verformen. Aufgrund der verwendeten Modulelemente mit unterschiedlichen elastischen Eigenschaften findet bei Krafteinwirkung ein sogenannter Kraftabbau statt, wobei die verbleibenden Kräfte über den Implantatpfosten aus biegefestem Werkstoff zum Drehpunkt im Bereich des Lastarmes des Implantatpfostens umgeleitet werden.

Es ist somit ein aus einem Primärzylinder und einem in diesem gehaltenen Sekundärzylinder bestehendes enossales Implantat mit einem Kraftleitungssystem geschaffen, mit dem eine Umleitung des Kraftflusses vom Krafteinleitungspunkt im Bereich des Zahnersatzes über das Kraftleitungssystem innerhalb des Sekundärzylinders, weiter über die ebenfalls Kraft umleitende Führungshülse und über den Primärzylinder in das knöcherne Implantatlager erfolgt, so dass neben einer Reduzierung der Belastungsspitzen und neben einer Vermeidung einer Überbelastung an der Austrittsstelle des Implantats aus dem Knochen der Drehpunkt des Implantatpfostens in den unteren Bereich des Implantats verlegt wird.

Weitere vorteilhafte Ausgestaltungen der Erfindung gehen aus den Unteransprüchen hervor.

Dadurch, dass der Implantatpfosten durch einen Thermoverschluss in der Führungshülse des Primärzylinders nach dem Einsetzen des Sekundärzylinders verankert ist, versetzen dynamische, an dem Implantatpfosten ansetzende Vertikal-, Horizontal- und Torsionskräfte den Implantatpfosten in Schwingungen, die in Wärme abgebaut werden, die in das Implantatinnere abgegeben wird. Nicht in Wärme umgesetzte, mengenmässig geringe mechanische Energien werden im Verschlusspunkt bzw. im Lagerbereich über den Primärzylinder an den Knochen abgegeben. Der Verschlusspunkt ist optimal in der Mitte der Vertikalachse des Primärzylinders plaziert. Die Schwingungsamplituden sind dabei insgesamt so ausgelegt, dass der Primärzylinder mechanisch nicht beansprucht wird.

Die Imitation des Parodontiums erfolgt durch kontrollierte Gleitverschiebung des Implantataufsatzes auf dem Primärzylinder, und zwar gedämpft durch das dauerelastische obere Modulrohr. Durch die Einbringung des Sekundärzylinders in den Primärzylinder unter definiertem Druck ist Spaltfreiheit durch Chemisorption im Bereich der Gleitzonen sichergestellt. Der Luftspalt oberhalb des Führungsrohres kann durch ein ein- oder aufgesetztes weiteres Modulrohr aus dauerelastischem Material ausgefüllt sein, das die Spaltdichtigkeit dann gewährleistet.

Es hat sich gezeigt, dass die Keramikoberteile, wie Schleimhautmanschette, bereits bei geringen Kräften von z.B. 5 kp zerbrechen können, was darauf zurückzuführen ist, dass die sphärische Fläche des Primärzylinders sich bei einem höheren Kraftaufwand keilförmig in das Keramikoberteil schiebt, so dass durch die dabei auftretende Keilwirkung es zu einem Bruch des Keramikoberteils kommen kann.

Auf die sphärischen Flächen kann jedoch nicht verzichtet werden, weil gerade der «Rotationseffekt» des Implantates seine universelle Verwendung sicherstellt. Hinzu kommt, dass das Schwingungsverhalten des zentralen Implantatpfostens das eines freischwingenden Stabes ist und der Implantatpfosten auch noch bei 250% der üblicherweise im Munde vorkommenden Horizontalkräfte im Implantat frei schwingt, also seine Dämpfungswirkung voll entfalten kann. Dabei wurde festgestellt, dass die Abdichtung des Keramikoberteils gegen das Keramikunterteil und die Gleiteigenschaften (Reibung) durch höhere Anpressung des Oberteils gegen das Unterteil nicht verändert wird und es hierzu lediglich einer geringfügigen Anpressung bedarf, um die erforderliche Abdichtung und das Gleiten sicherzustellen. Es war daher erforderlich, die auf den Implantataufsatz des Implantates einwirkenden Vertikalkräfte in dem Implantatboden und nicht auf dem Keramikoberteil abzufangen.

Im folgenden wird der Gegenstand der Erfindung in den Zeichnungen erläutert. Es zeigt

Fig. 1 teils in Ansicht, teils in einem senkrechten Schnitt ein aus einem Primärzylinder und einem Sekundärzylinder bestehendes enossales Implantat mit einem aus drei Modulrohren bestehenden Kraftleitungssystem,

Fig. 2 in einem senkrechten Schnitt den Primärzylinder nach Fig. 1,

Fig. 3 in einem senkrechten Schnitt den Sekundärzylinder nach Fig. 1 und

Fig. 4 in einem senkrechten Schnitt den Primärzylinder des Implantates mit als Kalotte ausgebildeter Gleitfläche.

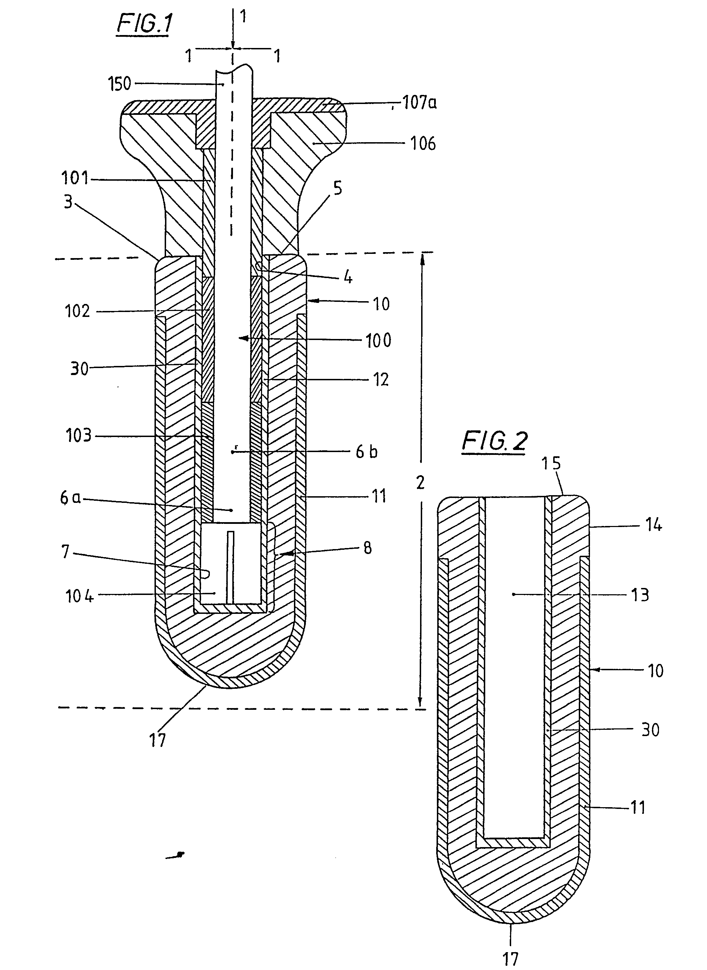

Das in Fig. 1 dargestellte enossale Implantat besteht aus einem Primärzylinder 10, dem sogenannten Aufnahmezylinder, und einem Sekundärzylinder 100, dem sogenannten Arbeitszylinder.

Der Primärzylinder 10 des enossalen Implantats besteht aus einem biegefesten Körper, insbesondere aus Aluminiumoxidkeramik und ist aussenseitig mit einer Hydroxylapatitkeramik beschichtet, die in Fig. 2 mit 11 bezeichnet ist. Dieser Primärzylinder 10 ist der eigentliche Implantatkörper bzw. Werkstoffträger und weist eine mittige Längsbohrung 13 auf, die den Innenraum bildet (Fig. 2).

Der Sekundärzylinder 100 wird nach der Implantation des Primärzylinders 10, d.h. etwa 3 Monate nach der Implantation, in die Längsbohrung 13 des Primärzylinders 10 eingesetzt, wodurch die Verbindung zwischen dem Implantat und dem Zahnersatz hergestellt wird.

In dem Innenraum bzw. in der Längsbohrung 13 des Primärzylinders 10 ist ein Führungsrohr 30 angeordnet, das sich in etwa über die gesamte Länge der Längsbohrung 13 erstreckt und u.a. das mühelose Einsetzen des Sekundärzylinders 100 in den Primärzylinder 10 ermöglicht.

Dieses Führungsrohr 30 gehört ebenfalls zum Kraftleitungssystem des enossalen Implantats, das, wie die nachstehend näher beschriebenen Modulelemente 101, 102, 103, 104 des Kraftleitungssystems, aus verschiedenen Werkstoffen bestehen kann. Durch die elastische Verformung des aus hierzu geeigneten Werkstoffen bestehenden Führungsrohres 30 in dem Primärzylinder 10 kann ein zusätzlicher Kraftabbau erreicht werden, wobei die verbleibenden Kräfte in kristallographisch bestimmte Richtungen, z.B. in die unteren Bereiche des Primärzylinders 10, umgeleitet werden können. Bei der Verwendung und Herstellung des Führungsrohres 30 haben die sogenannten Einkristalle des verwendeten Werkstoffes, z.B. schwingungsfähiges Metall, als elastisch verformbare Umhüllung des im Primärzylinder 10 lagernden Sekundärzylinders 100 eine grosse technische Bedeutung. Vor allem ist es möglich, einkristalline Werkstoffe mit vorgegebenen Fehlstellen zu züchten und zu verwenden, wodurch die elastische Verformung des Führungsrohres 30 steuerbar ist, z.B. im Zusammenhang mit der Kraftübertragung von dem Sekundärzylinder 100 auf den Primärzylinder 10.

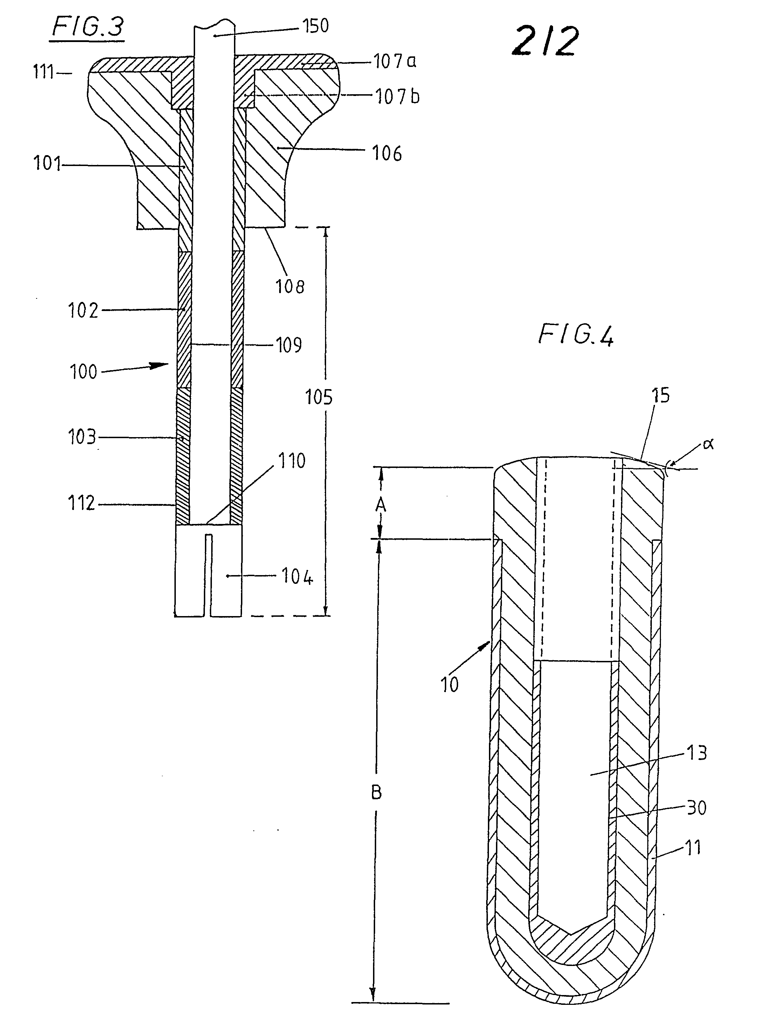

Der Sekundärzylinder 100 weist nach Fig. 3 einen in die Bohrung 13 des Primärzylinders 10 einsetzbaren, bei 105 angedeuteten Zapfen auf, so dass der Sekundärzylinder 100 auswechselbar ist. Der Durchmesser der zylindrischen Aussenwand 112 oder eines Teils der Aussenwand des Zapfens 105 des Sekundärzylinders 100 ist so viel grösser als der Durchmesser der Längsbohrung 13 des Primärzylinders 10 bemessen, dass der Zapfen 105 oder ein Teil des Zapfens z.B. 104 bei Körpertemperatur in der Längsbohrung 13 klemmend gehalten ist, jedoch bei einer Temperaturabsenkung aus der Längsbohrung des Primärzylinders lösbar bzw. herausziehbar ist. Eine solche Verbindung ist relativ einfach zu handhaben; sie kann sich nicht lösen und ist praktisch spaltfrei, so dass sich keine Bakterienherde bilden und Entzündungen entstehen können.

Der Sekundärzylinder 100 selbst ist von einem Implantatpfosten 150 gebildet, dessen oberes freies Ende eine in der Zeichnung nicht dargestellte lösbare Verschlussvorrichtung für den Zahnersatz trägt. Des weiteren umfasst der Sekundärzylinder 100 einen Implantataufsatz 106, der aus an sich bekannten Werkstoffen, wie z.B. Aluminiumoxidkeramik, besteht. Obenseitig kann der Implantataufsatz 106 mittels einer Deckplatte 107a abgedeckt sein, die mit einer nach innen gerichteten, halsartigen Verlängerung 107b versehen ist, die den Implantatpfosten 150 in seinem oberen Bereich umschliesst (Fig. 3).

Des weiteren ist in dem Sekundärzylinder 100 das sogenannte Kraftleitungssystem aus den Modulelementen 101 bis 104 untergebracht, das besonders für rotationssymmetrische zylindrische Implantate entsprechend Fig. 1 geeignet ist, die vorzugsweise aus Aluminiumoxidkeramik bestehen, jedoch auch für Implantate anderer Gestaltung bzw. Ausgestaltung Verwendung finden können. Dieses Kraftleitungssystem mit dem Implantatpfosten 150 ist ein Kraft übertragendes, materielles Bindeglied, das den Kraftfluss umleitet, und zwar vom Krafteinleitungspunkt in das knöcherne Implantatlager 2, und zwar derart, dass eine Reduzierung der Belastungsspitzen erreicht und eine Überbelastung an der Austrittsstelle 3 des enossalen Implantats aus dem Knochen vermieden wird (Fig. 1).

Dieses Kraft übertragende, materielle Bindeglied des Sekundärzylinders 100 (Fig. 3) besteht aus den Modulelementen 101 bis 104, welche rohr- oder ringförmig beispielsweise ausgebildet sein können und auf dem Implantatpfosten 150 aufgesetzt sind. Dieses Kraft übertragende, materielle Bindeglied kann auch über die Modulelemente 101 bis 104 hinaus auch noch das Führungsrohr 30 umfassen. Nach dem Einbringen des Sekundärzylinders 100 in die Längsbohrung 13 des Primärzylinders 10 wird der Sekundärzylinder 100 von dem Führungsrohr 30 umgeben und gehalten, welches sich in der Längsbohrung 13 des Primärzylinders 10 befindet bzw. dort angeordnet ist.

Das Kraftleitungssystem besteht aus den aufeinandergesetzten Modulelementen 101, 102, 103, 104, die in etwa gleiche Längen aufweisen können, jedoch mit unterschiedlichen elastischen Eigenschaften versehen sind (Fig. 1 und 3). Die Modulelemente können auch unterschiedliche Längen aufweisen.

Das untere Modulelement 103 mit oder ohne dem Teil 104 besteht aus einem unelastischen Werkstoff und ist in gleicher Weise wie der Implantatpfosten 150 starr ausgebildet. Auf dieses untere Modulelement 103 ist ein weiteres, das mittlere Modulelement 102 aufgesetzt, das aus einem Werkstoff mit einer geringen Elastizität gegenüber dem Modulelement 103 besteht. Das dritte und obere Modulelement 101 besteht aus einem stark elastischen Werkstoff. Das Modulelement kann beispielsweise auch aus einem vielkristallinen oder einkristallinen Werkstoff, aus einem Hartkunststoff oder einem anderen geeigneten Kunststoff mit entsprechenden elastischen Eigenschaften bestehen. Die anderen Modulelemente sind in Anpassung an die jeweils erforderliche Elastizität aus entsprechenden Werkstoffen hergestellt, wobei auch Kunststoffe, z.B. Silikon-Kautschuke, mit den unterschiedlichen Elastizitäts- und Härtegraden Verwendung finden können. Jedoch auch andere geeignete Werkstoffe können zur Anwendung gelangen, worauf nachstehend noch näher eingegangen wird.

Es besteht auch die Möglichkeit, die Modulelemente 101 bis 104 einstückig auszubilden, wobei dann das so erhaltene Kraftleitungssystem drei oder mehrere Bereiche mit unterschiedlichen elastischen Eigenschaften aufweist. Der untere Bereich ist dann unelastisch ausgebildet, der mittlere Bereich weist eine geringe Elastizität auf und der obere Bereich des Kraftleitungssystems ist mit einer hohen Elastizität versehen. Das Kraftleitungssystem erstreckt sich mit seinem oberen Modulelement bis in den Implantataufsatz 106 (Fig. 3).

Das Führungsrohr 30 kann ebenfalls bis in den Bereich des Implantataufsatzes 106 geführt sein, d.h. das Führungsrohr befindet sich auch im Implantataufsatz und wird bei der Montage des Sekundärzylinders 100 fest mit dem Implantataufsatz 106 verbunden. Das Führungsrohr 30 ist bodenseitig länger als der Implantataufsatz 106, so dass das Führungsrohr unterhalb der Auflagefläche des Implantataufsatzes etwa 1 mm bis 3 mm aus der Längsbohrung 13 des Primärzylinders 10 herausragt.

Das Führungsrohr 30 braucht jedoch nicht bis in den Implantataufsatz 106 geführt zu sein. Als Führungsstutzen dient dann das obere Modulelement 101, das in die Längsbohrung 13 des Primärzylinders 10 eingreift und mit dem Führungsrohr 30 im Primärzylinder 10 in metallischem Kontakt steht, der in Fig. 1 bei 4 angedeutet ist. Das Führungsrohr bringt die Auflageflächen von Primär- und Sekundärzylinder in absoluten Kontakt, zumal diese Auflageflächen 15, 108 des Primärzylinders 10 und des Sekundärzylinders 100 glanzpoliert sind, so dass ein dichter Abschluss 5 zwischen dem Primärzylinder 10 und dem Implantataufsatz 106 bzw. dem Sekundärzylinder 100 gegeben ist (Fig. 1 und Fig. 3).

Das unter Verwendung der Modulelemente erhaltene Kraftleitungssystem bewirkt die Umleitung einer an dem Implantatpfosten 150 angreifenden Kaumuskelkraft, die in Fig. 1 durch die Pfeile 1 angedeutet ist, und zwar z.B. eine horizontal einwirkende Kraft. Allerdings sollte der Implantatpfosten 150 des enossalen Implantats mit Nachbarzähnen oder Nachbarimplantaten durch einen geeigneten Zahnersatz verbunden sein, damit durch die Abstützung auf den Nachbarpfosten, z.B. Implantat oder Zahn, die Ursache einer Drehbewegung bzw. das sogenannte Drehmoment, ein Produkt aus Kraft mal Hebelarm in Bezug auf die Drehachse, ausgeglichen werden kann. In dieser Phase der Pfostenintegration wird die einwirkende Kraft, z.B. die Horizontalkraft, zu gleichen Teilen auf alle untereinander verbundenen Pfosten verteilt und der für das Kraftleitungssystem bzw. enossale Implantat verbleibende Anteil wird im Bereich des unteren Modulelementes 103, 104, bestehend aus einem unelastischen Werkstoff, z.B. aus einem Vielkristall, angreifen, und zwar im unteren Drittel des Primärzylinders 10. Dieses untere Modulelement 103, 104 besteht, wie der Implantatpfosten 150, aus einem spröden Werkstoff mit einem hohen Elastizitätsmodul. Dadurch ergibt sich eine gleichmässig verteilte, stark reduzierte Spannung im knöchernen Implantatlager 2 (Fig. 1).

Die elastischen Verformungseigenschaften der Modulelemente 101 bis 104 in ihren Eigenschaften als sogenannte Schwingungsdämpfer und die dadurch bedingte gleichmässige Bewegung des Implantatpfostens 150 um seine Ruhelage, bewirken eine Kraftumleitung, die mit dem Abbau von Spannungsspitzen einhergeht, besonders dann, wenn das untere Modulelement 103, 104 ein weitaus höheres Elastizitätsmodul als das darüberliegende Modulelement bzw. das obere Modulelement besitzt bzw. aus sehr sprödem Werkstoff besteht, so dass ein Teil der Wirkungskraft überwiegend in den kugelförmigen Boden 17 des enossalen Implantats umgeleitet wird (Fig. 1 und 2). Merkmal des Kraftleitungssystems ist der Implantatpfosten 150, der von verschiedenartigen Modulelementen 101 bis 104 umgeben ist, der sich unter Krafteinwirkung in seiner Form und Gestalt nicht verändert und sich nach der Montage des gesamten Systems überwiegend schwingend verhält, während sich die Modulelemente elastisch verformen.

Der Implantatpfosten 150 kann aus einem vielkristallinen Werkstoff, z.B. einem Metall mit einem hohen Elastizitätsmodul bestehen. Es hat sich gezeigt, dass die plastischen Eigenschaften des verwendeten metallischen Werkstoffes mit Ein- oder Vielkristallstruktur durch die Faktoren, die die Abweichungen des realen Gitters vom idealen Gitter verursachen, bestimmt werden. Hierbei handelt es sich insbesondere um die verschiedenen Arten von Gitterfehlstellen, die teils durch das Kristallwachstum bedingt sind, teils aber auch erst durch äussere Einwirkung, z.B. der Herstellung und Bearbeitung des Metalles, gebildet werden. Jeder Gitterfehler ist eine Komponente für das plastische Verhalten eines Werkstoffes unter der Wirkung von Kräften, die weit unterhalb der theoretischen Schubfestigkeit eines sogenannten Einkristalles liegen. Plastizität zeigen die meisten Metalle bzw. Kristalle. Wirken äussere Kräfte auf diese metallischen Körper, z.B. auf den Implantatpfosten 150, so ändern sie ihre Gestalt vor dem Eintritt des Bruches, bleibend, im Gegensatz zu den spröden Werkstoffen, z.B. Implantatpfosten 150, bei denen nach Überschreiten einer bestimmten Spannungsgrenze der Bruch eintritt.

Wird also bei einem plastisch verformten Körper die auf ihn wirkende Kraft weggenommen, so gehen die Verformungen nur zu einem geringen Teil zurück; zum grösseren Teil bleiben sie als sogenannte Formänderungen bestehen, während elastisch verformte Körper, z.B. der Implantatpfosten 150, beim Wegnehmen der Kraft die Verformungen zurückgeben. Der Implantatpfosten 150 besteht deshalb aus einem spröden Werkstoff. Die Stärke des Implantatpfostens 150 ist auf eine bestimmte Spannungsgrenze ausgerichtet, wobei die Kaumuskelkraft als äussere Kraft angenommen wird. Bedingt durch den Querschnitt und die Länge des Implantatpfostens 150 wird ein Überschreiten einer bestimmten Spannungsgrenze und somit der Bruch des Implantatpfostens verhindert, der unter diesen Bedingungen als Körper bei gleichmässiger Bewegung um seine Ruhelage schwingt.

Wird ein vielkristalliner Körper, z.B. der Implantatpfosten 150, durch eine Zugverformung plastisch gedehnt, so schnürt er sich allseitig gleichmässig ein; dagegen nimmt ein Einkristall, z.B. ein rohrförmiges Modulelement 101 bis 103 oder ein ringförmiges Modulelement, einen elliptischen Querschnitt ein. Es gleiten bei einem Einkristall, z.B. das dünne, schwingungsfähige Metallblech der Modulelemente 101 bis 104 und des Führungsrohres 30, kristallographisch bestimmte Ebenen des Gitters, die sogenannten Gleitebenen, in kristallographisch bestimmte Richtungen, den Gleitrichtungen, aufeinander ab. Zum Beispiel ein Vorgang, der bei einwirkender Kaumuskelkraft 1 auf den Implantatpfosten 150 und die Modulelemente 101 bis 104 sowie das Führungsrohr 30 in dem Primärzylinder 10 von Bedeutung ist. Es hat sich gezeigt, dass die Einkristalle als elastisch verformbare Umhüllung des Implantatpfostens 150, also das komplette Kraftleitungssystem aus den Modulelementen 101 bis 104, eine grosse technische Bedeutung haben. Das Kraftleitungssystem ist teilweise die elastisch verformbare Zwischenschicht zwischen dem Implantatpfosten 150 und dem Primärzylinder 10. Durch Verwendung geeigneter metallischer Werkstoffe, z.B. Einkristalle, mit vorgegebenen Fehlstellen und sogenannten Verunreinigungen sind die elastischen Verformungen der Modulelemente und des Führungsrohres 30 im Zusammenhang mit der Eigenbeweglichkeit der Zähne oder Intramobilität weiterer Implantate steuerbar. Ob und in welcher Grösse ein Kristall oder Werkstoff, aus dem die Modulelemente und das Führungsrohr bzw. die Führungsrohre bestehen, verformbar ist, hängt von den Faktoren ab, so z.B. von der Struktur, der Temperatur, der Verformungsart u.dgl., wobei anstelle eines einteiligen Führungsrohres auch ein mehrteiliges Führungsrohr verwendet werden kann.

Wird im Kraftleitungssystem ein Modulelement einer Kraft ausgesetzt, z.B. durch die Schwingungen des Implantatpfostens 150, so finden Formänderungen des Modulelementes statt, d.h. das dünne, schwingungsfähige Metall, aus dem das jeweilige Modulelement bestehen kann, wird elastisch verformt. Übersteigen bei der Verformung die auf die Modulelemente wirkenden Kräfte nicht die Grösse bzw. eine ganz bestimmte Grösse, so ist dieses eine elastische oder reversible Verformung. Wird dagegen die Elastizitätsgrenze überschritten, so findet entweder eine plastische Verformung statt oder der Werkstoff bzw. das Modulelement oder das Führungsrohr 30 bzw. der Primärzylinder des enossalen Implantats geht zu Bruch. Damit ist dargelegt, dass die elastischen Formänderungen oder Verformungen der Modulelemente 101 bis 104 von der Struktur des Werkstoffes abhängen. Neben einkristallinen Werkstoffen können auch vielkristalline Werkstoffe für die Herstellung der Modulelemente Verwendung finden.

Die Befestigung des Sekundärzylinders 100 im Innenraum bzw. in der Längsbohrung 13 des Primärzylinders 10 erfolgt mittels einer in an sich bekannter Weise ausgebildeten Einrichtung, die z.B. als Thermoverschluss ausgebildet sein kann, der in Fig. 3 bei 104 angedeutet ist. Zur Befestigung des Sekundärzylinders 100 können Fügeverbindungen 110, wie z.B. Stoffschluss mit anaeroben Kunststoffen oder andere geeignete Werkstoffe Verwendung finden.

Der Implantatpfosten 150 und das untere Modulelement 104 bestehen aus einem spröden Material mit einem hohen Elastizitätsmodul, und zwar beide aus Werkstoffen mit gleichen Elastizitätsmodulen. Bei Einwirkung einer Kraft 1 etwa im oberen Bereich des Zahnersatzes wirkt der Implantatpfosten 150 als Hebel. Der Hebeldrehpunkt liegt etwa im unteren Drittel des Primärzylinders 10, wenn beispielsweise das untere Modulelement und der Implantatpfosten 150 aus sprödem Material mit einem hohen Elastizitätsmodul bestehen und die oberhalb gelegenen Modulelemente 101, 102 bzw. 103 aus elastisch verformbaren Werkstoffen bestehen. Der im unteren Drittel des Primärzylinders 10 liegende Hebeldrehpunkt ist in Fig. 1 bei 6a angedeutet.

Dadurch, dass der Implantatpfosten 150 mit dem unteren Modulelement 104 bei 110 fest verbunden ist (Fig. 3), z.B. über Fügeverbindungen od.dgl. und beide wiederum in das Führungsrohr 30 in dem Primärzylinder 10 klemmend eingepasst sind, was in Fig. 1 bei 7 angedeutet ist, entsteht z.B. bei einer horizontalen Krafteinwirkung etwa im oberen Bereich des Zahnersatzes ein weiterer Hilfsdrehpunkt 6b. Wird der Implantatpfosten 150 durch die Krafteinwirkung beim Zahnersatz zur Störung gebracht, überträgt der Implantatpfosten 150 die Schwingungen auf das untere Modulelement, das aufgrund seiner festen Einpassung in dem Führungsrohr 30 des Primärzylinders 10 und des gleichen Elastizitätsmoduls wie der Implantatpfosten 150 insgesamt zu schwingen beginnt. Es entsteht dabei eine Vielzahl von Schwingungsfeldern, nämlich eines um den eigentlichen Hebeldrehpunkt 6a, eines um den Hilfsdrehpunkt 6b und weitere Schwingungsfelder im Gesamtbereich des unteren Modulelementes. Bewirkt wird dadurch, dass die Kraft vertikal nach unten verlagert und über das untere Modulelement 104 nicht über einen Punkt, sondern über die gesamte Länge des unteren Modulelementes an den Primärzylinder 10 und von diesem an den Knochen abgegeben wird.

Die Mitte des unteren Modulelementes 103 befindet sich im sogenannten Rotationszentrum 6b des Implantates und dieses wiederum in der Mitte des Primärzylinders 10. Über den Implantatpfosten 150 und die unteren Modulelemente 103, 104 findet somit eine Kraftverlagerung in vertikaler Richtung statt und gleichzeitig eine Kraftverteilung mit der Folge, dass eine im Kraftansatzpunkt 1 bzw. am Zahnersatz ansetzende Kraft im Kraftabgabebereich nur noch mit einem Teil der ursprünglichen Kraft zur Wirkung kommt (Fig. 1).

Das Führungsrohr 30, welches sich über einen grösseren Bereich der Länge des Primärzylinders 10 erstreckt, ist in dem Primärzylinder 10 mittels einer Klebverbindung fest angeordnet.

Bei dem in Fig. 4 gezeigten Ausführungsbeispiel des Implantats ist die Gleitfläche 15 des Primärzylinders 10 für den Implantataufsatz 106 als Kalotte mit einem Winkel $\alpha$ von insbesondere 9,27° ausgebildet.

Die Aussenbeschichtung 11 an dem Primärzylinder 10 besteht insbesondere aus einer Hydroxylapatitkeramik. Wie Fig. 4 zeigt, erstreckt sich diese Aussenbeschichtung 11 nur über einen Teilbereich des Primärzylinders 10, und zwar über den grösseren Bereich des Primärzylinders. Der obere Bereich des Primärzylinders ist aussenbeschichtungsfrei. Dieser aussenbeschichtungsfreie Abschnitt stellt die Kontaktzone A dar, die der Kortikalis entspricht, während die der Spongiosa entsprechende Kontaktzone B diese Aussenbeschichtung 11 trägt.

Die Auflagefläche 15 des Primärzylinders 10 für den Implantataufsatz 106 und der obere umlaufende Rand sind in einer Breite von 1 mm bis 5 mm hochglanzpoliert.

**Patentansprüche**

1. Enossales Implantat zur Befestigung von festsitzendem oder abnehmbarem Zahnersatz, bestehend aus zwei miteinander verbindbaren Teilen, von denen das eine Teil als in den Kieferknochen einbringbarer und sich in diesem kraftschlüssig verankernder Primärzylinder (10) mit einer mittigen Längsbohrung (13) und das andere Teil als Sekundärzylinder (100) ausgebildet ist, der einen in die Längsbohrung (13) des Primärzylinders einbringbaren und in diesem gehaltenen Implantatpfosten (150) zur Aufnahme des Zahnersatzes aufweist, und aus einem an dem Implantatpfosten (150) vorgesehenen Implantataufsatz (106), wobei der Implantatpfosten (150) an seinem freien oberen Ende für den Anschluss des Zahnersatzes ausgebildet ist und von einem die im Bereich des Zahnersatzes oder im Munde auftretenden Horizontal- und/oder Vertikal- und/oder Torsions-Kräfte und Schwingungen in den Sekundärzylinder (100) und von dort in den Primärzylinder (10) umleitenden Kraftleitungssystem aus mehreren, sich aneinanderreihenden Bereichen mit unterschiedlichen elastischen Eigenschaften umgeben und in dem Primärzylinder (10) fest oder lösbar gehalten ist, dadurch gekennzeichnet, dass der Implantatpfosten (150) als einarmiger Hebel in der Längsbohrung (13) des Primärzylinders (10) angeordnet ist und, zur Umleitung der im Bereich des Zahnersatzes oder im Munde auftretenden Horizontal- und/oder Vertikal- und/oder Torsions-Kräfte und Schwingungen in den bodenseitigen Bereich des Sekundärzylinders (100) und von dort in den Primärzylinder (10) oder in den Boden des Primärzylinders (10), das Kraftleitungssystem zur Verlagerung des Hebeldrehpunktes des Implantatpfostens (150) in das untere Drittel des Primärzylinders (10) aus mindestens einem bodenseitigen, unelastischen Modulelement (103; 104), einem mittleren, eine geringe Elastizität aufweisenden Modulelement (102) und einem oberen, eine hohe Elastizität aufweisenden Modulelement (101) besteht, das sich bis in den Implantataufsatz (106) erstreckt.

2. Enossales Implantat nach Anspruch 1, dadurch gekennzeichnet, dass der Implantatpfosten (150) in dem Primärzylinder (10) mittels eines Thermoverschlusses gehalten ist.

3. Enossales Implantat nach Anspruch 1 und 2, dadurch gekennzeichnet, dass in der Längsbohrung (13) des Primärzylinders (10) als elastisch verformbare Zwischenschicht ein Führungsrohr (30) angeordnet ist, das den Implantatpfosten (150) mit den diesen umgebenden Modulelementen (101, 102, 103; 104) aufnimmt.

4. Enossales Implantat nach den Ansprüchen 1 bis 3, dadurch gekennzeichnet, dass die Auflagefläche (15) des Primärzylinders (10) für den Implantataufsatz (106) und der obere umlaufende Rand (14) in einer Breite von 1 mm bis 5 mm hochglanzpoliert ist.

5. Enossales Implantat nach den Ansprüchen 1 bis 4, dadurch gekennzeichnet, dass die Auflagefläche (15) des Primärzylinders (10) für den Implantataufsatz (106) als Kalotte mit einem Winkel α von 9,27° ausgebildet ist.

6. Enossales Implantat nach den Ansprüchen 1 bis 5, dadurch gekennzeichnet, dass die der Kortikalis entsprechende Kontaktzone (A) des Primärzylinders (10) aussenbeschichtungsfrei ist, während die der Spongiosa entsprechende Kontaktzone (B) des Primärzylinders (10) eine Aussenbeschichtung (11) trägt.

7. Enossales Implantat nach Anspruch 6, dadurch gekennzeichnet, dass die Aussenbeschichtung (11) aus Hydroxylapatitkeramik besteht.

8. Enossales Implantat nach den Ansprüchen 3 bis 7, dadurch gekennzeichnet, dass der Implantatpfosten (150) des Sekundärzylinders (100) in seinem unteren Bereich mit dem Führungsrohr (30) mittels einer Klebeverbindung fest verbunden ist.

9. Enossales Implantat nach den Ansprüchen 1 bis 8, dadurch gekennzeichnet, dass der Primärzylinder (10) eine Aussenbeschichtung (11) aufweist.

10. Enossales Implantat nach Anspruch 9, dadurch gekennzeichnet, dass die Aussenbeschichtung aus Hydroxylapatitkeramik besteht.

11. Enossales Implantat nach den Ansprüchen 1 bis 10, dadurch gekennzeichnet, dass der Primärzylinder (10) aus Keramik, Titan oder anderen geeigneten Werkstoffen besteht.

**Revendications**

1. Implant endo-osseux pour la fixation d'une prothèse dentaire fixe ou enlevable, constitué par deux pièces pouvant être reliées l'une à l'autre, l'une de ces pièces étant configurée comme un cylindre primaire (10) pouvant être mis en place dans le maxillaire et s'ancrant à celui-ci par adhérence et présentant un alésage longitudinal central (13), et l'autre pièce étant configurée comme un cylindre secondaire (100) qui présente un montant d'implant (150) pour loger la prothèse dentaire, pouvant être mis en place dans l'alésage longitudinal (13) du cylindre primaire et étant maintenu dans celui-ci, et par une pièce de dessus d'implant (106) prévue sur le montant d'implant (150), le montant d'implant (150) étant configuré à son extrémité supérieure libre pour le raccord de la prothèse dentaire et étant entouré par un système de transmission de forces, composé de plusieurs zones à la file l'une de l'autre avec différentes propriétés élastiques, qui dérive les forces horizontales et/ou verticales et/ou de torsion et les oscillations, qui interviennent dans la zone de la prothèse dentaire ou dans la bouche, dans le cylindre secondaire (100) et, de là, dans le cylindre primaire (10), et maintenu dans le cylindre primaire (10) de manière fixe ou amovible, caractérisé en ce que le montant d'implant (150) est placé comme levier à bras unique dans l'alésage longitudinal (13) du cylindre primaire (10) et que, pour la dérivation des forces horizontales et/ou verticales et/ou de torsion et les oscillations, qui interviennent dans la zone de la prothèse dentaire ou dans la bouche, dans la zone du fond du cylindre secondaire (100) et, de là, dans le cylindre primaire (10) ou dans le fond du cylindre primaire (10), le système de transmission de forces pour le déplacement du point d'appui du levier du montant d'implant (150) dans le tiers inférieur du cylindre primaire (10) est constitué par au moins un élément modulaire (103; 104) non élastique, situé du côté du fond, un élément modulaire central (102) présentant une faible élasticité et un élément modulaire supérieur (101) présentant une grande élasticité, qui s'étend jusque dans la pièce de dessus d'implant (106).

2. Implant endo-osseux selon la revendication 1, caractérisé en ce que le montant d'implant (150) est maintenu dans le cylindre primaire (10) au moyen d'une fermeture thermique.

3. Implant endo-osseux selon les revendications 1 et 2, caractérisé en ce qu'un tube de guidage (30), qui loge le montant d'implant (150) et les éléments modulaires (101, 102, 103; 104) qui l'entourent, est placé comme couche intermédiaire déformable dans l'alésage longitudinal (13) du cylindre primaire (10).

4. Implant endo-osseux selon les revendications 1 à 3, caractérisé en ce que la surface d'appui (15) du cylindre primaire (10) du montant d'implant (106) et le bord périphérique supérieur (14) sont polis spéculaire, ce dernier sur une largeur d'1 à 5 mm.

5. Implant endo-osseux selon les revendications 1 à 4, caractérisé en ce que la surface d'appui (15) du cylindre primaire (10) du montant d'implant (106) est formé comme une calotte avec un angle α de 9,27°.

6. Implant endo-osseux selon les revendications 1 à 5, caractérisé en ce que la zone de contact (A) du cylindre primaire (10), qui correspond au cortex, est exempte de revêtement extérieur, tandis que la zone de contact (B) du cylindre primaire (10), qui correspond au tissu spongieux, porte un revêtement extérieur (11).

7. Implant endo-osseux selon la revendication 6, caractérisé en ce que le revêtement extérieur (11) est constitué par de la céramique d'apatite hydroxylée.

8. Implant endo-osseux selon les revendications 3 à 7, caractérisé en ce que le montant d'implant (150) du cylindre secondaire (100) est relié dans sa zone inférieure au tube de guidage (30) de manière fixe au moyen d'un assemblage collé.

9. Implant endo-osseux selon les revendications 1 à 8, caractérisé en ce que le cylindre primaire (10) présente un revêtement extérieur (11).

10. Implant endo-osseux selon la revendication 9, caractérisé en ce que le revêtement extérieur est constitué par de la céramique d'apatite hydroxylée.

11. Implant endo-osseux selon les revendications 1 à 10, caractérisé en ce que le cylindre primaire (10) est constitué par de la céramique, du titane ou d'autres matières appropriées.

**Claims**

1. Endosseous implant for securing fixed or removable dentures, consisting of two interconnectable parts, of which the one part is constructed as a primary cylinder (10) provided with a longitudinal bore (13) and insertable into the jaw bone and anchoring itself in a nonpositive fashion within the same, and the other part as a secondary cylinder (100) which is provided with an implant pillar (150) for accommodating the denture that is insertable into the longitudinal bore (13) of the primary cylinder and retained within the latter, and of an implant fixture (106) provided on the implant pillar (150), in which the implant pillar (150), at its free upper end, is constructed so as to be connected with the denture and surrounded by a force transmission system which redirects the horizontal and/or vertical and/or torsional forces and vibrations occurring within the area of the denture or in the mouth into the secondary cylinder (100) and, from there, into the primary cylinder (10), said system comprising several areas in side-by-side arrangements and possessing differing elastic properties and retained fixedly or detachably within the primary cylinder (10), characterized in that the implant pillar (150) is arranged as one-armed lever within the longitudinal bore (13) of the primary cylinder (10) and, in order to redirect the horizontal and/or vertical and/or torsional forces and vibrations occurring within the area of the denture or in the mouth into the bottom area of the secondary cylinder (100) and, from there, into the primary cylinder (10) or into the bottom of the primary cylinder (10), while the force transmission system for displacing the lever fulcrum of the implant pillar (150) into the bottom third of the primary cylinder (10), consists of at least one bottom inelastic modular element (103; 104), one central modular element possessing a slight degree of elasticity (102) and an upper modular element (101) possessing a high degree of elasticity which extends as far as into the implant fixture (106).

2. Endosseous implant according to the Claim 1, characterized in that the implant pillar (150) within the primary cylinder is retained by means of a thermoclosure.

3. Endosseous implant according to the Claims 1 and 2, characterized in that, in the longitudinal bore (13) of the primary cylinder (10), a guide tube (30) is disposed as elastically deformable intermediate layer which receives the implant pillar (150) with the modular elements (101, 102, 103; 104) surrounding the same.

4. Endosseous implant according to the Claims 1 to 3, characterized in that the bearing surface (15) of the primary cylinder (10) for the implant fixture (106) and the upper circumferential rim (14) are mirror-finished to a width of from 1 mm to 5 mm.

5. Endosseous implant according to the Claims 1 to 4, characterized in that the bearing surface (15) of the primary cylinder (10) for the implant fixture (106) is constructed in a cap-shaped fashion having an angle α of 9,27°.

6. Endosseous implant according to the Claims 1 to 5, characterized in that the contact zone (A) of the primary cylinder (10) which corresponds to the corticalis, is devoid of an external coating, whereas the contact zone (B) of the primary cylinder (10), which corresponds to the spongiosa, is provided with an external coating (11).

7. Endosseous implant according to the Claim 6, characterized in that the external coating (11) consists of hydroxylapatite ceramics.

8. Endosseous implant according to the Claims 3 to 7, characterized in that the implant pillar (150) of the secondary cylinder (100), within its lower area, is rigidly connected to the guide tube (30) by means of a bonding connection.

9. Endosseous implant according to the Claims 1 to 8, characterized in that the primary cylinder (10) is provided with an external coating (11).

10. Endosseous implant according to the Claim 9, characterized in that the external coating (11) consists of hydroxylapatite ceramics.

11. Endosseous implant according to the Claims 1 to 10, characterized in that the primary cylinder (10) consists of ceramics, titanium or other suitable materials.

# 1/2

FIG.1

FIG.2

FIG.3

FIG.4

212